# EUROPÄISCHE PATENTSCHRIFT

(11) **EP 1 139 924 B1**
(45) Veröffentlichungstag und Bekanntmachung des Hinweises auf die Patenterteilung: **14.02.2007**
(21) Anmeldenummer: 99900043.3
(22) Anmeldetag: 12.01.1999
(51) Int. Cl.: A61F 2/30

(54) **OBERFLÄCHENSTRUKTUR FÜR EIN ENOSSALES IMPLANTAT**
SURFACE STRUCTURE FOR INTRA-OSSEOUS IMPLANT
STRUCTURE SUPERFICIELLE POUR IMPLANT INTRAOSSEUX

(43) Veröffentlichungstag der Anmeldung: 10.10.2001
(73) Patentinhaber: Lipat Consulting AG, 4051 Basel (CH)
(72) Erfinder: JOOS, Ulrich, D-48147 Münster (DE)
(74) Vertreter: Heinen, Detlef
(86) Internationale Anmeldenummer: PCT/CH1999/000010
(87) Internationale Veröffentlichungsnummer: WO 1999/013700

(56) Entgegenhaltungen:
- EP-A- 0 388 576
- EP-A- 0 639 356
- EP-A- 0 669 116
- WO-A-95/09583
- WO-A-97/28760
- WO-A-97/46179
- GB-A- 2 045 083
- US-A- 4 865 603
- US-A- 4 865 608

## Beschreibung

### Anwendungsgebiet der Erfindung

Die Erfindung betrifft ein in Knochen, vorzugsweise in Humanknochen, operativ einzubringendes Implantat und umfasst z.B. die Gebiete der Osteosynthese, der Prothetik - im Bereich von Hand-, Fuss-, Hüft und Extremitätenchirurgie - sowie maxillofaciale und dentale Implantologie. Speziell befasst sich die Erfindung mit dem Design des Implantats und dessen Oberfläche zur Erzielung einer sofortigen Primärstabilität und einer raschen Osseointegration.

### Stand der Technik

Bei der Stabilität eines Implantats stellen sich zwei Anforderungen:
a) eine möglichst unmittelbare postoperative Belastbarkeit, d.h. die sofortige Primärstabilität, welche allein auf mechanischen Gesetzen beruht; und
b) eine möglichst rasche Sekundärstabilität durch Osseointegration, die biologischen Gesetzen folgt.

Man hat erkannt, dass - abgesehen von der Qualität der Implantation und der örtlichen Bedingungen im Umfeld des Implantatlagers - für die Primärstabilität des Implantats dessen Design von ausschlaggebender Bedeutung ist und ein äusseres Schraubengewinde die besten Resultate für eine sofortige Stabilität liefert. Die Osseointegration hingegen ist entscheidend von der Materialauswahl - hier kommen zunehmend Titan und Titanbasislegierungen sowie porzellanähnliche Substanzen zur Anwendung - und der Oberflächenbeschaffenheit des Implantats abhängig.

In der GB-A-2 045 083 wird eine Oberflächenrauhigkeit in der Grössenordnung von 0,01 µm bis 0,3 µm empfohlen. Die EP-B-0 388 576 geht von einer idealen Implantatoberfläche mit einer Makrorauhigkeit von mehr als 10 µm, vorzugsweise mehr als 20 µm aus, der eine Mikrorauhigkeit in der Grössenordnung von 2 µm und feiner überlagert ist. Zur Erzeugung der Makrorauhigkeit wird die Behandlung der Implantatoberfläche mit einem Strahlmittel und zur Erzeugung der Mikrorauhigkeit das Ätzen in Säure vorgeschlagen. Aus der WO-A-97 28760 ist für Osteosyntheseschrauben ein Rauhigkeitswert Rₜ von ca. ≥ 10 µm (maximum peak-to-valley height) bekannt. Nach SCHROEDER, A.; SUTTER, F.; BUSER, D.; KREKELER, G. (Orale Implantologie. Georg Thieme Verlag Stuttgart, 2. Aufl. 1994, S. 48 ff.), wird zur Oberflächenvergrösserung und Förderung der Osseointegration auf die Implantatoberfläche eine Plasmabeschichtung mit einer Schichtdicke von ca. 20-40 µm und einer Rauhtiefe von ca. 15 µm aufgebracht. Insgesamt vertritt die Fachwelt die verfestigte Auffassung, dass Makro- und Mikrorauhigkeiten, besonders in Kombination, die Verankerung von enossalen Implantaten im Knochen begünstigen.

Aus der US-A-4,865,603 sind prothetische Implantate bekannt, welche eine Oberfläche mit einer Struktur aufweisen. Die Struktur umfasst erste und zweite Vertiefungen, wobei die Dimensionen der zweiten Vertiefungen im Bereich von 25% bis 75% der Dimensionen der ersten Vertiefungen liegen. Die Dimensionen der ersten Vertiefungen, insbesondere die Breite, liegen im Bereich zwischen 200 µm und 1000 µm, bevorzugt zwischen 300 µm und 700 µm, folglich liegen die Dimensionen der zweiten Vertiefungen im Bereich von 50 µm bis 750 µm, bevorzugt im Bereich von 75 µm bis 525 µm. Die Tiefe sowohl der ersten Vertiefungen als auch der zweiten Vertiefungen liegt grundsätzlich im Bereich der Dimension, insbesondere der Breite, der jeweiligen Vertiefungen. Die zweiten Vertiefungen können von ihrer Gestalt her rillenförmig oder diamantförmig ausgebildet sein.

Die bisherigen Massnahmen brachten_zwar partielle Fortschritte für die primäre und sekundäre Verankerung von enossalen Implantaten im knöchemen Implantatlager, jedoch sind weitere Verbesserungen wünschenswert, damit die Erfolgsquote der Implantate erhöht wird, sich das gesamte chirurgische Prozedere verkürzt und somit der versorgte Patient weniger in Anspruch genommen wird und rascher eine zumindest annähernd übliche Lebensform erlangen kann.

### Aufgabe der Erfindung

Angesichts der vorgegebenen Zielstellung liegt der Erfindung die Aufgabe zugrunde, für enossale Implantate eine Oberflächenstruktur zu schaffen, welche unmittelbar postoperativ eine hohe Primärstabilität bewirkt und eine rasche, möglichst intensive biologische Osseointegration des eingesetzten Implantats begünstigt.

### Übersicht über die Erfindung

Die essentiellen Erfindungsmerkmale lassen sich wie folgt zusammenfassen: Das zum Einsetzen in einen Knochen bestimmte enossale Implantat ist auf der Oberfläche mit einer spezifischen Rauhigkeit versehen, wobei die Implantatoberfläche eine Vielzahl von Vertiefungen mit einer vertikal gemessenen Tiefe von 20 µm bis 60 µm aufweist, zwischen denen Plateauflächen liegen. Am Grund der Vertiefungen liegen Bodenflächen, und von den Bodenflächen zu den Plateauflächen erstrecken sich Seitenflächen. Die Bodenflächen und die Plateauflächen weisen eine Horizontalausdehnung von jeweils 10 µm bis 20 µm auf. Die Vertiefungen sowie die Plateauflächen sind glatt, d.h. sie weisen keine Oberflächenrauhigkeit auf, die unterhalb der zellulären Dimension von osteogenetischen Zellen, also unterhalb von 10 µm, liegt.

Die Seitenflächen der Vertiefungen nehmen vorzugsweise einen zur Horizontalen gemessenen Neigungswinkel von maximal etwa 30° ein. Das Implantat weist bevorzugt eine obere Halspartie auf und unterhalb dieser oberen Halspartie ein selbstschneidendes Gewinde. Die Halspartie des Implantats kann mit einer porzellanähnlichen Beschichtung versehen sein. Die Oberflächenenergie, das sogenannte Zeta-Potential, und die Oberflächenspannung des Implantats werden vorzugsweise möglichst gering gehalten. Als Material für die Implantate verwendet man vorzugsweise Titan, Titanbasislegierungen oder porzellanähnliche Substanzen. Die Vertiefungen sind vorzugsweise als systematisch, zueinander parallel verlaufende, trapezförmige Nuten ausgebildet, welche mechanisch erzeugt sind.

Dank der Erfindung steht nun ein Implantat zur Verfügung, das sich bei einer soliden unmittelbar postoperativen Primärstabilität infolge begünstigter Osseointegration - im Wege des biologischen Verwachsens des Knochens mit der Implantatoberfläche - durch eine beschleunigte und erhöhte Belastbarkeit, bezüglich der zeitlich sekundären Verankerung, auszeichnet.

### Kurzbeschreibung der beigefügten Zeichnung

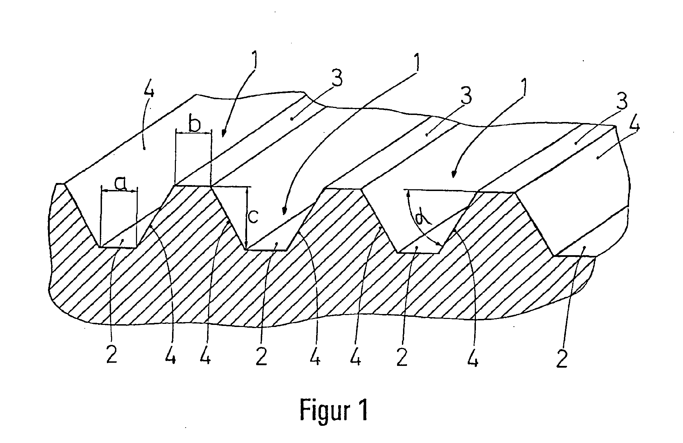

- Figur 1 zeigt:: eine schematische Schnittdarstellung durch die erfindungsgemässe Oberflächenstruktur eines enossalen Implantats.

### Detaillierte Beschreibung der Erfindung

Für zeitlichen Verlauf und Intensität der Osseointegration ist die Oberflächenbeschaffenheit der eingesetzten Implantatmaterialien von entscheidendem Einfluss, denn das Implantat findet seinen Kontakt zum Implantatlager über die Implantatoberfläche, welche somit auch für die Entwicklung dieses Kontakts und dessen Erhaltung massgeblich ist. Angesichts der Bedeutung der Implantatoberfläche für die knöcherne Integration wurden verschiedene Oberflächenstrukturen vorgeschlagen, um ein Maximum an verankertem Kontakt zwischen dem das eingesetzte Implantat umgebenden Knochen und der Implantatoberfläche zu erreichen. Umfangreiche und vielfach betriebene Forschungen auf der Gewebeebene führten zu der Erkenntnis, dass mit einer Rauhigkeit versehene Implantatoberflächen einen vermehrten Knochenkontakt aufweisen, als dies bei glatten Oberflächen der Fall ist.

Hingegen hat man sich bisher kaum mit der Reaktion osteogenetischer Zellen auf den unterschiedlichen Oberflächenmikrostrukturen beschäftigt. Insbesondere sind die bisherigen Untersuchungen über die ursächlichen molekularen Zusammenhänge zwischen dem biologischen Zellverhalten in Relation zu verschiedenen Oberflächenstrukturen nur sehr dürftig. Da vornehmlich die molekularen Faktoren die Grundlage zellulärer Tätigkeit und der Gewebereaktion bilden, basiert die Erfindung auf Untersuchungen in dieser Richtung. In einem neugeschaffenen Knochenimplantatlager zirkulieren Proteine und Ionen, die über die Zellreaktionen der ersten Minuten und Stunden entscheiden. Die spätere Zellreaktion wird ferner durch die Toxizitätseinwirkung des implantierten Werkstoffs beeinflusst. Die Dauerreaktion der Lagerzellen ist auf das Vorhandensein eines implantierten Materials eine Antwort auf die Kombination des molekularen Reaktionspotentials des Materials und seiner toxischen Einwirkung.

In Versuchsserien wurde ein markanter Unterschied in der Zellreaktion auf glattpolierten im Vergleich zu rauhen (korundgestrahlten und plasmabeschichteten) Werkstoffoberflächen festgestellt. Auf einer glatten Oberfläche stellt sich ein höheres Zellwachstum mit flachen ausgebreiteten Zellen ein als auf einer rauhen Implantatoberfläche. Bei einer glatten Oberfläche wird mehr Fibronektin adsorbiert, das sich breitflächig verteilt. Auf rauhen Oberflächen variiert die Fibronektinadsorption von spärlich bis nicht nachweisbar. Die Zellen auf glatten Proben weisen Filopodien und Lamellopodien in multiple Richtungen auf, während eine grosse Anzahl der Zellen auf einer rauhen Oberfläche von spindelförmiger Morphologie ist. Der Einfluss der Oberflächenstruktur von Titan auf das Verhalten von Fibroblasten bei Ausbreitung, Wachstum und Bildung von "focal adhesions" war bereits früherer Untersuchungsgegenstand (vgl. Könönen M., et al: "Effect of surface processing on the attachment, orientation, and proliferation of human gingival fibroblasts on titanium". J Biomed Mater Res (26) 1325-1341,1992). Hiernach adhärieren und wachsen auch Fibroblasten eher auf glatten Oberflächen als auf rauhen. "Focal adhesions" wurden lediglich in Verbindung mit glatten Oberflächen beobachtet. Auch bei Chondrozyten wurden Abflachungs- und Wachstumsverhältnisse bei den glatten Oberflächen ermittelt, welche denen im Vergleich zu rauhen Oberflächen überlegen sind.

Die Zellphänomenologie ist naturgemäss materialspezifisch, aber darüber hinaus wird sie durch die Oberflächenmikrostruktur bestimmt. Stahl und Titan sind zu einer vergleichbaren Bindung von Fibronektin fähig. Damit übereinstimmend zeigten Untersuchungsergebnisse, dass in der Zellkultur die Verteilung von Fibronektin und das Zellwachstum auf glatten Stahl- und Titanoberflächen vergleichbar ist. Auf rauhen Titanoberflächen stellte sich die entsprechende Fibronektinbindung und das Zellwachstum weniger und spärlicher ein. In Korrelation mit der Fibronektinverteilung auf den unterschiedlich gestalteten Titanoberflächen war die Zellausbreitung ausgeprägter und die Monolayerentwicklung fortgeschrittener auf glatten als auf rauhen Oberflächen.

Diese Ergebnisse führen zu der Hypothese, dass die Diskrepanz im Zellverhalten bei glatten im Vergleich zu rauhen Titanoberflächen auf das topomorphologische Muster der Oberfläche unterhalb 10 µm zurückzuführen ist, wobei 10 µm den Grössenordnungswert der Zelldimension darstellt. Nähert sich eine Zelle einer glatten Oberfläche, so ist jeder Punkt potentiell für einen "focal contact" zugänglich. Bei einer rauhen Oberfläche hingegen ist ein Zellkontakt nur an den herausragenden Arealen möglich. Mangelt es an Arealen für die Bildung von "focal contacts", so bleiben Zelladhäsion und -ausbreitung aus.

In den Versuchsserien zeigte sich, dass die rauhesten Oberflächen (plasmabeschichtet und korundgestrahlt und geätzt) mit den kleinsten Mengen adsorbierten Fibronektins einhergehen; vermutlich wird Fibronektin nur an den für die Zellen begrenzt verfügbaren Stellen adsorbiert. Dagegen waren grössere Flächen an den glatteren Oberflächen für die Adsorption der nachgewiesenen grösseren Fibronektinmengen verfügbar. Zum zweiten war die Zellmigration ausgedehnter bei rauhen als bei den glatten Oberflächen. Dies lässt sich insbesondere von dem Bewegungsmodus der Zellen ableiten. Zellen, die lange Strecken migrieren, schicken lange explorative Filopodien voraus und sind bipolar, während sich langsam fortbewegende Zellen eine multipolare Erscheinung haben.

Es wurde beobachtet, dass die Bildung von Fibronektinmikrofilamenten nur bei polierten Stahl- und Titanoberflächen nachweisbar eintritt; auf rauheren Oberflächen war Aktin nur schwach oder nicht nachweisbar vorhanden. Dies führt zur Deutung, dass, wenn "focal adhesions" auf rauhen Oberflächen gebildet werden, diese ungenügende Stabilität besitzen, um die Aktinfaserbildung zu unterstützen. Ein Vergleich zwischen den hiesigen Erkenntnissen und den früheren Studien ist von grundlegender Bedeutung. Die vorherigen Untersuchungen ergaben hierbei, dass Fibroblasten bevorzugter auf glatten Titanoberflächen adhärieren und wachsen als auf rauhen; "Focal adhesions" wurden lediglich in Verbindung mit glatten Oberflächen beobachtet (vgl. Könönen M., et al, a.a.O.). An glatten Oberflächen - gegenüber rauhen - stellen sich bei Chondrozyten auch überlegene Abflachungs- und Wachstumsverhältnisse ein.

Nach den der Erfindung den Weg bereitenden Untersuchungen eröffnet sich eine Erklärung für das Verhalten der Fibroblasten und Chondrozyten sowie dafür, dass sich Aufrauhungen im Zelldimensionsbereich nachteilig auf die Zelladhäsion auswirken. Hingegen eine im Zelldimensionsbereich möglichst glatte Implantatoberfläche begünstigt die Osseointegration.

Die Zellphänomenologie, d.h. Adhäsion, Ausbreitung und Bildung von Verknüpfungsstellen an Substraten, haben weitreichende Bedeutung für die Zellfunktion und naheliegend auch für die knöcherne Integration der Implantate. Die Übertragung von Information auf das Zellinnere wird über Zell-Substratverknüpfungen abgewickelt. Dabei dienen "focal contacts" der Fortbewegung und "focal adhesions" der Abflachung. "Focal contacts" sind geradlinige Strukturen mit der Dimension 0,25-0,5 x 2-10 µm, die nach 4 bis 20 Minuten aufgelöst sind. "Focal adhesions" sind strukturell und zeitlich stabiler sowie räumlich ausgedehnter; sie sind Voraussetzung für die Synthese und Ausscheidung extrazellulärer Matrix (vgl. Woods A., et al: "Stages in specialization of fibroblast adhesion and deposition of extracellurar matrix." Eur J Cell Biol (32) 108-116, 1983). Erst durch die Abflachung treten die Zellen in die DNA-Synthese ein; Zellabrundung aber hebt DNA-Synthese auf.

Auf der Zellseite der "focal adhesions" erfolgt - über Vinculin, Talin und cx-Aktinin - die Organisation des zytoskelettalen Aktins in Bündeln (stress fibres). Hierdurch geschieht vor allem die Signalübertragung (signal transduction) von der Unterlage an das Zellinnere zur Synthese extrazellulärer Matrix. Der Substratum-Fokaladhäsion-Komplex reguliert dann über Polyribosome und MRNA auch die Rate der Proteinsynthese und die Absonderung gewebespezifischer Proteine.

Diesen Prozessen - also der Oberflächenmikrogestalt - kommt für eine optimale Einheilung und Gewebeintegration von Knochenimplantaten folglich erhebliche Bedeutung zu. Je mehr die chemischen Eigenschaften und die Mikrogestaltung der Oberfläche eines Werkstoffs die Bildung von "focal adhesions" und "focal contacts" erlauben, desto intensiver wird seine biologische Integration (Osseointegration) begünstigt. Grundsätzlich ist eine mikromorphologisch rauhe Knochenimplantatoberfläche anzustreben, da sich somit die Oberfläche und der potentielle Kontakt zwischen Knochen und Implantat beträchtlich erhöhen. Eine vergrösserte Oberfläche ergibt zwar auch vermehrte Freisetzung von Zerfallspartikeln, die gewonnene Flächenvergrösserung erhöht jedoch das Interaktionspotential und die mechanische Stabilität. Die Aufrauhungen sollen jedoch den Dimensionsbereich der Zelle nicht unterschreiten.

Es gibt zellphysikalische und molekulare Gründe, warum Unebenenheiten im Dimensionsbereich der Zelle für die Gewebeintegration nachteilig wirken dürften. Eine Zelle kann sich nicht in jeden Schlupfwinkel einarbeiten, um überall eine enge Beziehung zum Substrat beizubehalten, jedoch ist der enge physikalische Zusammenhang zwischen Zelle und Substrat unerlässlich für die Morphogenese. Ferner führen Mikroerhebungen bzw. -vertiefungen in diesem Bereich zu einer Verminderung bei der Zellanhaftung und -adhäsion, wenn sie der Geometrie und Mindestdimension zur Bildung von "focal adhesions" und "focal contacts" nicht genügen.

Damit erklärt sich auch die nicht optimale Osseoeintegration von plasmabeschichteten Implantatwerkstoffen, wo die Plasmaschicht Inseln verhältnismässig glatter Struktur aufweist, die die Bildung von "focal adhesions" und "focal contacts" nicht mindern sollte. Jedoch ist die Oberfläche insgesamt grösstenteils uneben und zeigt somit insbesondere im Zelldimensionsbereich ungünstige Verhältnisse für die Bildung von "focal adhesions" und "focal contacts". Die zumeist angewendete Säureätzung der Implantatoberfläche ergibt eine Oberflächenporosität, wo Werkstoffsubstanz zumindest bei Titan bevorzugt an Korngrenzen und an definierten kristallographischen Orientierungen abgetragen werden dürfte. Da die Ätzung auch intrakristallin angreift, entstehen Porositäten auch hier im Zelldimensionsbereich, was sich bei der Bildung von "focal adhesions" und "focal contacts" nachteilig bemerkbar machen dürfte. Korundstrahlung alleine bewirkt keine ausgeprägten Mikroporositäten an der Materialoberfläche. Schliesst man der Korundstrahlung eine Säureätzung an, so entstehen Mikroeinbuchtungen mit einem Durchmesser von ca.10 nm bis 100 nm. Diese Grössen liegen aber ausserhalb des Zelldimensionsbereichs und beeinträchtigen die Bildung von "focal adhesions" und "focal contacts" nicht negativ, vorausgesetzt die Mikrokrater sind in sich glatt. Wie nur säuregeätzte stellen sich jedoch auch zunächst nur korundgestrahlte und dann säuregeätzte Oberflächen im Zelldimensionsbereich als rauh dar und sind deshalb für die Zellanhaftung ebenso ungünstig.

Zellmechanisch betrachtet kann sich eine Zelle, die sich auf einer Strecke bewegt, nicht um eine Abwinkelung von mehr als 32°. Vermutlich können die Aktinfasern, die zur Zellbewegung am Vorderrand der Zelle in den "focal contacts" ansetzen und die übrige Zelle nachziehen, nur direkt und quasi nicht um die Ecke ziehen (vgl. Dunn, G.A.; Heath J.P.: "A new hypothesis of contact guidance in tissue cells". Exp Cell Res (101) 1-14, 1976). Die Zelle kann dann die Bewegung in diese Richtung verweigern oder bei gegebenen Bedingungen den Abhang mit Zellkörpern bzw. Ausläufern überbrücken. In Versuchsserien wurde dieses Überbrückungsverhalten bei polierten und geätzten, plasmabeschichteten sowie bei korundgestrahlten und geätzten Oberflächen festgestellt. Bei unpolierten Calciumphosphat-Keramiken, jedoch mit nicht ausgeprägten Reliefveränderungen bei Dimensionen im subzellulären Bereich, erfolgt die Zellausbreitung ebenfalls.

Dass eine Zelle nicht jede unebene Fläche überdecken kann, dürfte auch mit der Geometrie und Mindestdimension des Zellverankerungsapparats zusammenhängen. Zellen benötigen "focal contacts" für ihre Fortbewegung (locomotion) bzw. "focal adhesions" für ihre Verankerung und Ausbreitung. Ein für die Fortbewegung erforderlicher "focal contact" ist 0,25 µm bis 0,5 µm breit und mindestens 2 µm lang. Vermutlich erlauben Unterlageunebenheiten kleinerer Dimension die Bildung dieser wichtigen Verankerungsstukturen nicht. Eine Zelle verweilt deshalb nicht an einer zu klein dimensionierten Stelle, sondern verlässt sie hin zu einer grösser dimensionierten Fläche.

Unter der Prämisse, dass Osteoblasten bei ihrer morphogenetischen Tätigkeit unter Einfluss den zuvor diskutierten molekularen und zellulären Gesetzmässigkeiten stehen, müsste dies zwangsläufig auch Auswirkungen auf die Entstehung von Interface-Knochen, und damit auf die Osseointegration haben. Die Oberflächenchemie und die Oberflächenmikromorphologie dürften die wesentlichen Werkstoffparameter darstellen. Implantatoberflächen, welche die massgeblichen Moleküle der Knochenheilung und -morphogenese an sich binden können, versprechen einen grösseren Implantaterfolg, insbesondere im Langzeitbereich. Den Anforderungen sowohl der spezifischen Oberfläche als auch der Interface-Zellaktivität entsprechend gilt daher eine Oberflächenmikromorphologie mit Vertiefungen von 20 µm bis etwa 60 µm. Ebene Zonen sollten eine Horizontalausdehnung von 10 µm bis etwa 20 µm aufweisen. Die Vertiefungen sind idealerweise möglichst glatt und Winkelungen an der Implantatoberfläche betragen maximal etwa 30°. Die Oberflächenenergie (Zeta-Potential) und die Oberflächenspannung sollte möglichst gering gehalten werden. Am Implantathals wäre eine porzellanähnliche Beschichtung ideal.

### Bevorzugtes Ausführungsbeispiel

Anhand der beigefügten Figur 1 folgt nun die Beschreibung eines bevorzugten Ausführungsbeispiels. Die Implantatoberfläche weist eine Vielzahl von Vertiefungen **1** auf. Am Grund der Vertiefungen **1** liegen Bodenflächen **2** und zwischen den Vertiefungen **1** erstrecken sich Plateauflächen **3.** Von den Bodenflächen **2** zu den Plateauflächen **3** verlaufen aufwärts ansteigende Seitenflächen **4.** Die Vertiefungen **1** besitzen eine vertikal zwischen den Plateau- und Bodenflächen **3,2** gemessene Tiefe **c** von etwa 20 µm bis etwa 60 µm. Die Bodenflächen **2** verfügen über eine Horizontalausdehnung **a,** welche etwa 10 µm bis etwa 20 µm in der Breite beträgt. Die Horizontalausdehnung **b** der Plateauflächen **3** beträgt in der Breite ebenfalls etwa 10 µm bis etwa 20 µm. Die Boden- und Plateauflächen **2,3** sind in ihrer Länge endlos, wenn die Vertiefungen **1** radial um das Implantat umlaufen. Die Vertiefungen **1** sowie die Platauflächen **3** sind idealerweise durch Polieren möglichst glatt, weisen also keine Oberflächenrauhigkeit unterhalb der zellulären Dimension auf.

Die Seitenflächen **4** der Vertiefungen **1** nehmen einen zur Horizontalen gemessenen Neigungswinkel α von maximal etwa 30° ein. Vorzugsweise sind die Vertiefungen **1** als systematisch, zueinander parallel verlaufende, trapezförmige Nuten ausgebildet und mechanisch durch Zerspanen oder Einprägen erzeugt.

## Patentansprüche

1. Enossales Implantat mit einer Implantatoberfläche, die zur Begünstigung der Belastbarkeit des Implantats mit einer spezifischen Rauhigkeit versehen ist, wobei die Implantatoberfläche eine Vielzahl von Vertiefungen **(1)** mit einer vertikal gemessenen Tiefe **(c)** von 20 µm bis 60 µm aufweist, zwischen denen Plateauflächen **(3)** liegen,
wobei am Grund der Vertiefungen **(1)** Bodenflächen **(2)** liegen und sich von den Bodenflächen **(2)** zu den Plateauflächen **(3)** Seitenflächen **(4)** erstrecken, **dadurch gekennzeichnet, dass** die Bodenflächen (**2**) und die Plateauflächen (**3**) eine Horizontalausdehnung **(a,b)** von jeweils 10 µm bis 20 µm aufweisen,
und dass die Vertiefungen (**1**) sowie die Plateauflächen (**3**) glatt sind, d.h. keine Oberflächenrauhigkeit aufweisen, die unterhalb der zellulären Dimension von osteogenetischen Zellen, also unterhalb von 10 µm, liegt.

2. Implantat nach Anspruch 1, **dadurch gekennzeichnet, dass** die Seitenflächen **(4)** der Vertiefungen **(1)** einen zur Horizontalen gemessenen Neigungswinkel (α) von maximal etwa 30° einnehmen.

3. Implantat nach Anspruch 1 oder 2, **dadurch gekennzeichnet, dass** das Implantat eine obere Halspartie aufweist und unterhalb dieser oberen Halspartie ein selbstschneidendes Gewinde.

4. Implantat nach einem der Ansprüche 1 bis 3, **dadurch gekennzeichnet, dass** das Implantat aus Titan oder einer Titanbasislegierung besteht.

5. Implantat nach einem der Ansprüche 1 bis 4, **dadurch gekennzeichnet, dass** die Vertiefungen **(1)** systematisch, zueinander parallel verlaufende, trapezförmige Nuten sind mit dazwischen liegenden Plateauflächen.

## Claims

1. An intraosseous implant having an implant surface which is provided with a specific roughness in order to promote the load-bearing capacity of the implant, the implant surface having a multiplicity of depressions (1) having a vertically measured depth (c) of from 20 µm to 60 µm with plateau faces (3) being arranged between them, wherein at the bottom of the depressions (1) base faces (2) are arranged and wherein side faces (4) extend from the base faces (2) to the plateau faces (3),
**characterized in that** the base faces (2) and the plateau faces (3) each have a horizontal extent (a,b) of from 10 µm to 20 µm,
and **in that** the depressions (1) and the plateau faces (3) are smooth, that is to say they have no surface roughness which is below the cellular dimension of osteogenetic cells, i.e. below 10 µm.

2. Implant according to claim 1, **characterized in that** the side faces (4) of the depressions (1) form an angle (α) of inclination relative to the horizontal of about 30° maximum.

3. Implant according to claim 1 or claim 2,
**characterized in that** the implant has an upper neck portion with a self-tapping thread being provided below this upper neck portion.

4. Implant according to any one of claims 1 to 3, wherein the implant is made of titanium or a titanium-based alloy.

5. Implant according to any one of claims 1 to 4, wherein the depressions (1) are systematically arranged trapezoid grooves extending parallel to one another, with plateau faces lying between them.

## Revendications

1. Implant intraosseux avec une surface d'implant qui est dotée d'une rugosité spécifique afin de favoriser l'aptitude de l'implant à supporter les charges, dans lequel la surface de l'implant présente une pluralité de creux (1), ayant une profondeur (c) mesurée verticalement de 20 µm à 60 µm, entre lesquels se trouvent des surfaces de plateaux (3),
dans lequel des surfaces de fond (2) se trouvent au fond des creux (1) et des surfaces latérales (4) s'étendent depuis les surfaces de fond (2) jusqu'aux surfaces de plateaux (3),
**caractérisé en ce que** les surfaces de fond (2) et les surfaces de plateaux (3) présentent respectivement une étendue horizontale (a, b) allant de 10 µm à 20 µm,
et **en ce que** les creux (1) ainsi que les surfaces de plateaux (3) sont lisses, c'est-à-dire ne présentent aucune rugosité de surface inférieure à la dimension des cellules ostéogènes, c'est-à-dire inférieure à 10 µm.

2. Implant selon la revendication 1, **caractérisé en ce que** les surfaces latérales (4) des creux (1) présentent un angle d'inclinaison (α) d'environ 30° au maximum par rapport à l'horizontale.

3. Implant selon la revendication 1 ou la revendication 2, **caractérisé en ce que** l'implant présente une partie de collet supérieure et, en dessous de cette partie de collet supérieure, un filetage autotaraudeur.

4. Implant selon l'une quelconque des revendications 1 à 3, **caractérisé en ce que** l'implant est en titane ou un alliage à base de titane.

5. Implant selon l'une quelconque des revendications 1 à 4, **caractérisé en ce que** les creux (1) sont des rainures trapézoïdales qui s'étendent systématiquement parallèlement les unes aux autres, avec des surfaces de plateaux situées entre elles.
